# EUROPÄISCHE PATENTSCHRIFT

(11) **EP 0 965 324 B1**
(45) Veröffentlichungstag und Bekanntmachung des Hinweises auf die Patenterteilung: **22.10.2003**
(21) Anmeldenummer: 99110868.9
(22) Anmeldetag: 07.06.1999
(51) Int. Cl.: A61K 7/13

(54) **Haarfärbemittel und Verfahren zu dessen Herstellung**
Hair dye composition and process for producing same
Composition pour la teinture des cheveux et procédé pour sa préparation

(30) Priorität: 19.06.1998 DE 19827434; 19.05.1999 DE 19922851
(43) Veröffentlichungstag der Anmeldung: 22.12.1999
(73) Patentinhaber: KPSS-Kao Professional Salon Services GmbH, 64297 Darmstadt (DE)
(72) Erfinder: Fröhling, Beate, Dr., 64625 Bensheim (DE); Golinski, Frank, Dr., 64297 Darmstadt (DE)

(56) Entgegenhaltungen:
- WO-A-92/01438
- WO-A-94/01076

## Beschreibung

Die vorliegende Erfindung betrifft Haarfärbemittel auf Basis einer feinen wäßrigen Emulsion, enthaltend mindestens ein Oxidationsfarbstoff-Vorprodukt, mit verbesserter Viskositätsstabilität, die durch Vermischen bei Raumtemperatur herstellbar ist.

Permanente Haarfärbemittel auf Basis von Oxidationsfarbstoffen erfreuen sich einer weiten Verbreitung. Ihre Anwendung erfolgt in der Regel dergestalt, daß eine flüssige, zumeist als wäßrige Emulsion vorliegende, mindestens ein Oxidationsfarbstoff-Vorprodukt, im allgemeinen mindestens eine Entwickler- und mindestens eine Kupplersubstanz, enthaltende Zusammensetzung unmittelbar vor der Anwendung mit einer Peroxid enthaltenden Zusammensetzung vermischt, und die Mischung auf das Haar aufgebracht wird.

WO-A-92 01438 beschreibt Haarfärbemittel, bestehend aus einer fließfähigen, wäßrigen Oxidationsfarbstoffzubereitung (A) und einer fließfähigen wäßrigen Oxidationsfarbstoffzubereitung (B), die unmittelbar von dem Aufbringen auf das Haar zu einem gelförmigen Färbenansatz gemischt werden. Sie enthalten eine flüssige Fettsäure, ein C₂-C₆ Polyol, ein Anlagerungsprodukt von 1 bis 5 Mol Ethylenoxid an einem C₁₂-C₂₂-Fettalkohol und/oder ein Anlagerungsprodukt von 1 bis 4 Mol Ethylenoxid an ein lineares Fettalkylamin mit 12 bis 22 C-Atomen.

Dabei ist die Einhaltung gewisser Viskositätsverhältnisse zweckmäßig. Es hat sich jedoch gezeigt, daß diese Emulsionen häufig thixotrope Eigenschaften aufweisen, d.h., bei der Lagerung in der Dose oder Tube ihre Viskosität erhöhen, was dann bei der Applikation und der Vermischung mit der Peroxid-Zusammensetzung Probleme bereitet. Darüber hinaus müssen diese Emulsionen durch Vermischen der Phasen bei erhöhter Temperatur hergestellt werden., was einen hohen Zeit - und Energieaufwand bedingt.

Die Erfindung geht daher von der Aufgabenstellung aus, diese Probleme zu vermeiden und feine stabile Haarfärbeemulsionen zur Verfügung zu stellen, die sich bei Raumtemperatur herstellen lassen und aufgrund ihrer Feinheit auch eine gute Vermischbarkeit mit dem Oxidationsmittel, d.h. im Regelfall Wasserstoffperoxid, gewährleisten, was wiederum zu einer gleichmäßigen Verteilung des fertigen Färbemittels auf dem Haar und damit zu einer guten Färbeleistung führt.

Erfindungsgemäß wurde nämlich festgestellt, daß stabile, feine, wäßrige Haarfarbe-Emulsionen, die mindestens ein Oxidationsfarbstoff-Vorprodukt enthalten, dann erhalten werden, wenn sie ein Gemisch aus
a) 2,5 bis 10 Gew.-% mindestens einer C₁₀-C₁₈-Fettsäure,
b) 5 bis 25 Gew.-% mindestens eines C₁₀-C₂₂-Fettalkoholethoxylats, und
c) 5 bis 30 % Gew.-% mindestens eines flüssigen Zuckerfettsäureesters, jeweils berechnet auf die Gesamtzusammen-setzung der Emulsion, enthalten.

Gemäß einer bevorzugten Ausführungsform der Erfindung wird als Fettsäure Ölsäure eingesetzt, doch ist auch die Verwendung anderer Fettsäuren wie Laurinsäure, Myristinsäure, Linolsäure, Linolinensäure und Fettsäuregemischen wie Cocosfettsäure möglich. Der Schmelzpunkt dieser Fettsäuren sollte vorzugsweise unter 50 °C liegen.

Die bevorzugte Menge einer C₁₂-C₁₈- Fettsäure liegt bei etwa 3 bis 8, insbesondere bei etwa 5 Gew.-%, berechnet auf die Gesamtzusammensetzung.

Als C₁₀-C₂₂-Fettalkoholethoxylat werden vorzugsweise Lauryl-, Kokos- und Oleylalkohol-Ethylenoxid-Kondensate in einer Menge von etwa 5 bis 25, insbesondere etwa 10 bis 20, beispielsweise etwa 15 Gew.-%, berechnet auf die Gesamtzusammensetzung, eingesetzt.

Bevorzugt enthält das Haarfärbemittel den Bestanteil (c) in einer Menge von 10 bis 25 gew.-%, berechnet auf die gesamtzusammensetzung.

Bevorzugte flüssige Zuckerfettsäureester sind Saccharose- und Glucosediester, insbesondere Dioleate, insbesondere glucosediolate Von diesen sind wiederum Glucose- und Methylglucosedioleat in einer Menge von etwa 10 bis 25, vor allem etwa 15 Gew.-% der Gesamtzusammensetzung, besonders geeignet.

Das bevorzugte Gewichtsverhältnis der Komponenten a) zu c) liegt bei 1:5 bis 1:1, insbesondere etwa 1:2 bis 1:3, dabei wird eine in ihren Eigenschaften optimale Haarfärbung erhalten.

Die Herstellung der erfindungsgemäßen wäßrigen Haarfärbeemulsion erfolgt vorzugsweise durch Zusammenrühren von etwa 20 bis 40 Gewichtsteilen der Ölphase, enthaltend mindestens eine C₁₀-C₁₈-Fettsäure, mindestens ein C₁₀-C₂₂-Fettalkoholethoxylat und mindestens einen flüssigen Zuckerfettsäureester mit etwa 80 bis 60 Gewichtsteilen einer wäßrigen Phase, enthaltend mindestens ein Oxidationsfarbstoff-Vorprodukt und mindestens ein wasserlösliches Tensid, bei etwa 15 bis etwa 35 °C, vorzugsweise bei Raumtemperatur, d.h. etwa 20 bis 25 °C.

Die Viskosität der erfindungsgemäßen Haarfärbeemulsion liegt vorzugsweise bei etwa 10.000 und 30.000, insbesondere bei etwa 15.000 bis 25.000, beispielsweise bei etwa 20.000 mPa•s, gemessen bei 20 °C im Brookfield-Viskosimeter RVT.

Die Wasserphase kann wasserlösliche Emulgatoren enthalten. Als solche können insbesondere anionische Tenside verwendet werden.

Geeignete anionaktive Tenside im Rahmen der Erfindung sind insbesondere in einer Menge von etwa 0,25 bis etwa 5 Gew.-%, vorzugsweise etwa 0,4 bis 2,5 Gew.-% der Gesamtzusammensetzung (einsatzfertige Emulsion) enthalten.

Dabei handelt es um solche vom Sulfat-, Sulfonat-, Carboxylat- und Alkylphospht-Typ, vor allem natürlich diejenigen, die in Haarbehandlungsmitteln üblicherweise zum Einsatz gelangen, insbesondere die bekannten C₁₀-C₁₈-Alkylsulfate und die entsprechenden Ethersulfate, beispielsweise C₁₂-C₁₄-Alkylethersulfat, Laurylethersulfat, insbesondere mit 1 bis 4 Ethylenoxidgruppen im Molekül, Acylaminocarbonsäuren wie Lauroylsarkosinat und -glutamat, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettiger Mono- und Dialkylphosphate, die milde, hautverträgliche Detergentien darstellen.

Im Rahmen der Erfindung weiterhin geeignete anionische Tenside sind α-Olefinsulfonate bzw. deren Salze und Alkalisalze von Sulfobersteinsäurehalbestern, beispielsweise das Dinatriumsaiz des Monooctylsulfosuccinats, und Alkalisalze langkettiger Monoalkylethoxysulfosuccinate.

Geeignete Tenside vom Carboxylat-Typ sind Alkylpolyethercarbonsäuren und deren Salze der Formel

R - (C₂H₄O)ₙ - O - CH₂ COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel 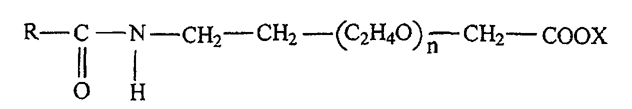 worin R und X die vorstehend angegebene Bedeutung haben und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO®" und "AKYPO-SOFT®".

Auch C₈-C₂₀-Acylisethionate können, allein oder im Gemisch mit anderen Tensiden, eingesetzt werden, ebenso Sulfofettsäuren und deren Ester.

Gegebenenfalls können auch amphotere bzw. zwitterionische Tenside als wasserlösliche Emulgatoren eingesetzt werden, insbesondere im Gemisch mit anionaktiven Tensiden, wobei die Gesamtmenge vorzugsweise bei etwa 0,25 bis 5, insbesondere etwa 0,5 bis 2,5 Gew.-% der Färbeemulsion liegen soll.

Als solche sind insbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren wie Cocoaminoacetat, Cocoaminopropionat und Natriumcocoamphopropionat und -acetat haben sich als geeignet erwiesen.

Im einzelnen könne Betaine der Struktur 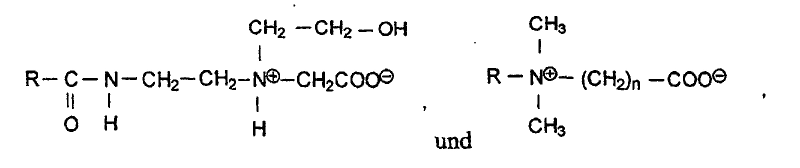 wobei R eine C₈-C₁₈-Alkylgruppe und n 1 bis 3 bedeuten,

Sulfobetaine der Struktur 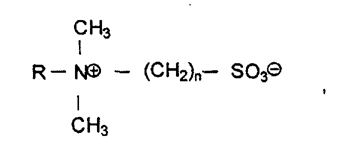 Wobei R eine C₈-C₁₈-Alkylgruppe und n 1 bis 3 bedeuten,
und Amidoalkylbetaine der Struktur 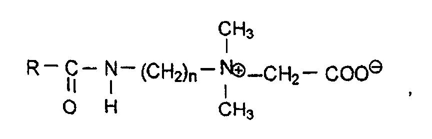 wobei R eine C₈-C₁₈-Alkylgruppe und n 1 bis 3 bedeuten, eingesetzt werden.

Auch die Verwendung nichtionischer wasserlöslicher Tenside, z.B. von C₈-C₁₈-Alkylpolyglucosiden mit einem Polymerisationsgrad von 1 bis 5, ist in den genannten Mengen alleine oder im Gemisch mit anionischen und/oder amphoteren bzw. zwitterionischen oberflächenativen Substanzen möglich.

Auch Aminoxide und Fettsäuremono- und -dialkanolamide sind einsetzbar.

Weitere Tenside sind auch kationische Tenside wie die bekannten quaternären Ammoniumverbindungen mit einer oder zwei Alkyl- bzw. Alkenylgruppen mit 10 bis 22 Kohlenstoffatomen im Molekül, insbesondere in einer Menge von 0,1 bis 7,5, vorzugsweise 0,25 bis 5, besonders bevorzugt 0,5 bis 2,5 Gew.-%, der Gesamtzusammensetzung.

Geeignete langkettige quaternäre Ammoniumverbindungen, die allein oder im Gemisch miteinander eingesetzt werden können, sind insbesondere Cetyltrimethylammoniumchlorid, Dimethyldicetylammoniumchlorid, Trimethylcetylamoniumbromid, Behenyltrimoniumchlorid, Stearyltrimethylammoniumchlorid, Dimethylstearybenzylammoniumchlorid, Benzyltetradecyldimethylammoniumchlorid, Dimethyl- dihydriertes- Talgammoniumchlorid, Laurylpyridiniumchlorid, Lauryldimethylbenzylammoniumchlorid, Lauryltrimethylammoniumchlorid, Tris-(oligooxyethyl)alkylammoniumphosphat, Cetylpyridiniumchlorid, etc.

Grundsätzlich sind alle quaternären Ammoniumverbindungen geeignet, die im CTFA International Cosmetic Ingredient Dictionary unter der Bezeichnung "Quaternium" aufgeführt sind.

Die erfindungsgemäße Haarfärbeemulsion enthält mindestens ein Oxidationsfarbstoffvorprodukt, zweckmäßigerweise ein Gemisch aus mindestens einer Entwickler- und mindestens einer Kupplersubstanz.

Diese sind an sich bekannt und beispielsweise in der Monographie von K. Schrader, Grundlagen und Rezepturen der Kosmetika, 2. Aufl. (1989), S. 784-799, beschrieben.

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 2-(2'-Hydroxyethylamino)-5-aminotoluol und 1-Amino-4-bis-(2'hydroxyethyl)-aminobenzol, bzw. deren wasserlösliche Salze; beispielhafte Kupplersubstanzen sind Resorcin, 2-Methylresorcin, 4-Chlorresorcin, 2-Amino-4-chlorphenol, 4-(N-Methyl)aminophenol, 2-Aminophenol, 3-Aminophenol, 1-Methyl-2-hydroxy-4-aminobenzol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodiphenylamin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, 1,3-Diaminotoluol, α-Naphthol, 1,4-Diamino-2-chlorbenzol, 4,6-Dichlorresorcin, 4-Hydroxy-1,2-methyldioxybenzol, 1,5-Dihydroxynaphthalin, 1,7-Dihydroxynaphthalin, 2,7-Dihydroxynaphthalin, 1-Hydroxynaphthalin, 4-Hydroxy-1,2-methylendioxybenzol, 2,4-Diamino-3-chlorphenol, und/oder 1-Methoxy-2-amino-4-(2'-hydroxyethylamino)-benzol, ohne daß diese beispielhafte Aufzählung Anspruch auf Vollständigkeit erheben könnte.

Entwickler- und Kupplersubstanzen sind vorzugsweise im Molverhältnis 1:3 bis 5:1, insbesondere etwa 1:1 und etwa 3:1, enthalten; ihr Anteil in der erfindungsgemäßen Farbemulsion kann jeweils etwa 0,1 bis etwa 5 Gew.-%, je nach gewünschter Färbung, betragen.

Dies Oxidationsfarbstoffvorprodukte sind zweckmäßigerweise bereits in der Phase enthalten; sie können jedoch auch, falls gewünscht, dem Fertigprodukt zusammen mit der Ölphase oder im Anschluß daran zugesetzt werden.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5%, insbesondere 0,1 bis 1 % Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, Stabilisatoren, Fett und Öle, Verdickungsmittel, Komplexbildner, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind.

Die erfindungsgemäß hergestellten Färbeemulsionen weisen vorzugsweise einen im alkalischen Bereich liegenden pH-Wert, insbesondere zwischen 8 und etwa 12,5, vorzugsweise zwischen 8,5 und 11, auf.

Gemäß einer weiteren bevorzugten Ausführungsform enthalten die erfindungsgemäßen Haarfärbeemulsionen noch 0,005 bis 1 Gew.-%, berechnet auf die, Gesamtzusammensetzung der Emulsion, mindestens einer wasserunlöslichen Substanz, ausgewählt aus Titandioxid, Zinkoxid, Talkum, Aluminiumoxid, Wismutoxidchlorid, Calciumcarbonat, Magnesiumcarbonat, Calciumsulfat, Calciumphosphaten, Bariumsulfat, Aluminiumsilikat und/oder Alkalialuminiumsilikat.

Bevorgugt enthält das Haarfärbemittel 0,01 bis 0,5 gew.-%, berechnet auf die, gesamtzusammensetzung, mindestens einer wasserunlöslichen Substanz.

Dadurch wird die zuweilen auftretende Gelbildung und die möglicherweise darauf beruhende Verfärbung beim Vermischen der Färbeemulsion mit der Oxidationsmittel-Zusammensetzung vermieden.

Die genannten Substanzen weisen vorzugsweise eine mittlere Teilchengröße (Teilchendurchmesser) im Mikro- bzw. Nanometerbereich von weniger als 100 µm, beispielsweise unterhalb 10 µm, insbesondere weniger als 1 µm, auf.

Besonders bevorzugt sind ultrafeine Teilchen mit einem Durchmesser zwischen etwa 10 und etwa 500 nm, beispielsweise 30 bis 300 nm.

Zur Verbesserung der Dispergierbarkeit der Teilchen in den Emulsionen können diese mit organischen Materialien beschichtet sein, beispielsweise mit Organopolysiloxanen oder Phospholipiden wie Lecithin, wie es aus der EP-A 446 290 oder der GB-A 2 184 356 bekannt ist.

Als geeignete Substanzen sind insbesondere Titandioxid, Zinkoxid und Talkum, z.B. "Mearltalkum", zu nennen.

Der Anteil dieser Substanzen liegt bei 0,005 bis 1, vorzugsweise 0,01 bis 0,5, insbesondere 0,05 bis 0,25 Gew.-%, berechnet auf die Gesamtzusammensetzung der Färbeemulsion.

Der Zusatz der wasserunlöslichen Substanzteilchen kann dabei in jedem Stadium der Herstellung, vorzugsweise durch Zugabe zur Wasserphase, beispielsweise bei etwa 15 bis 75°C, insbesondere etwa 20 bis 35°C, erfolgen.

Zur Applikation wird die erfindungsgemäße Oxidations-Vorprodukt-Emulsion mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemittels, d.h. nach Vermischen mit Peroxid, kann sowohl im schwach sauren, d.h. einem Bereich von 5,5 bis 6,9, im neutralen als auch im alkalischen Bereich, d.h. zwischen pH 7,1 und 10 liegen.

Im folgenden wird die Erfindung anhand von Ausführungsbeispielen erläutert.

### Beispiel 1

Als wäßrige Phase wurde die folgende Zusammensetzung verwendet:

| | |
|---|---|
| Ammoniak, 25 %ig | 13,50 Gew.-% |
| Pyrogenes Siliciumdioxid | 0,15 |
| Natriumlaurylsulfat | 1,50 |
| EDTA (Trinatriumsalz) | 0,30 |
| Ammoniumchlorid | 0,70 |
| Natriumsulfit | 1,50 |
| Ascorbinsäure | 0,30 |
| Kationisches Pflanzeneiweißhydrolysat | 0,75 |
| Panthenol | 0,90 |
| Hopfenextrakt | 0,75 |
| Parfum | 0,60 |
| p-Toluylendiaminsulfat | 0,80 |
| Resorcin | 0,07 |
| 4-Chlorresorcin | 0,25 |
| 3-Aminophenol | 0,03 |
| Wasser | ad 100,00 |

In 68 Gewichtsteile dieser Zusammensetzung wurden bei 20 bis 25 °C jeweils 32 Gewichtsteile der folgenden Ölphase mit einer Rührgeschwindigkeit von etwa 9.000 bis 10.000 U/min während 1 bis 2 Minuten gerührt.

| **Zusammensetzung Nr.** | **1** | **2** | **3** | **4** | **5** | **6** |
|---|---|---|---|---|---|---|
| Ölsäure | 31,2 | 30,0 | - | 60,0 | 60,0 | 15,5 |
| Laureth-2 | 37,5 | - | - | 40,0 | - | - |
| Oleth-5 | - | 30,0 | 30,0 | - | 40,0 | 38,0 |
| Methylglucosediolat (in Gew.-%) | 31,3 | 40,0 | 70,0 | - | - | 46,5 |

Die Zusammensetzung Nr. 1, 2 und 6 stellen feine, stabile Emulsionen dar, die sich ausgezeichnet mit 6 %-iger H₂O₂-Lösung vermischen lassen und eine intensive, stabile, langanhaltende Mittelblondfärbung des Haares ergeben.

Die Zusammensetzung Nr. 4 und 5 ergaben keine Emulsion, sondern gelartige, schwer handhabbare Produkte; Zusammensetzung Nr. 3 stellte ein dünnflüssiges, unbrauchbares Produkt dar.

### Beispiel 2

Als wäßrige Phase wurde die folgende Zusammensetzung verwendet:

| | |
|---|---|
| Ammoniak, 25%-ig | 13,50 (Gew.-%) |
| Pyrogenes Siliciumdioxid | 0,15 |
| Natriumlaurylsulfat | 1,50 |
| EDTA (Trinatriumsalz) | 0,30 |
| Ammoniumchlorid | 0,70 |
| Natriumsulfit | 1,50 |
| Ascorbinsäure | 0,30 |
| Kationisches Pflanzeneiweißhydrolysat | 0,75 |
| Panthenol | 0,90 |
| Hopfenextrakt | 0,75 |
| Parfum | 0,60 |
| p-Toluylendiaminsulfat | 0,80 |
| Resorcin | 0,07 |
| 4-Chlorresorcin | 0,25 |
| 3-Aminophenol | 0,03 |
| Wasser | ad 100,00 |

In 68 Gewichtsteile dieser Zusammensetzung wurden bei 55 °C 0,25 Gewichtsteile mikrofeines Titandioxid (P25 der Degussa) und 32 Gewichtsteile der folgenden Ölphase mit einer Rührgeschwindigkeit von etwa 9.000 bis 10.000 U/min während 1 bis 2 Minuten eingerührt.

| **Ölphase**: | |
|---|---|
| Ölsäure | 31,2 |
| Laureth-2 | 37,5 |
| Methylglucosediolat (in Gew.-%) | 31,3 |

Es wurde eine feine Emulsion erhalten, die sich ausgezeichnet mit 6%-iger H₂O₂-Lösung vermischen ließ und eine intensive, stabile, lang anhaltende Mittelblondfärbung des Haares ergab, wobei beim Anrühren keinerlei Gelbildung oder Verfärbung auftrat.

Daraus ergibt sich der überraschende Vorteil der erfindungsgemäßen Zusammensetzungen.

### Beispiel 3

Der Ersatz des Titandioxids durch 0,1 Gewichtsteile Zinkoxid (mittlerer Teilchendurchmesser 50 bis 120 nm) ergab eine Haarfärbeemulsion mit gleicher Wirkung.

### Beispiel 4

720 Gewichtsteile einer Wasserphase, enthaltend

| | |
|---|---|
| Ammoniak, 25%-ig | 92,00 (Gew.-Teile) |
| Pyrogenes Silica | 1,00 |
| Natriumlaurylsulfat | 10,00 |
| Komplexbildner (EDTA) | 2,00 |
| Ammoniumchlorid | 5,00 |
| Natriumsulfit | 10,00 |
| Ascorbinsäure | 2,00 |
| Hopfenextrakt | 5,00 |
| Kationisiertes Pflanzenproteinhydrolysat | 5,00 |
| Panthenol | 6,00 |
| Parfum | 3,50 |
| p-Toluylendiaminsulfat | 5,40 |
| Resorcin | 0,50 |
| 4-Chlorresorcin | 1,60 |
| 3-Aminophenol | 0,20 |
| 2,4,5-Triamino-6-hydroxypyrimidinsulfat | 0,10 |
| Wasser | 520,70 |

wurden bei 30 bis 35°C und einer Rührgeschwindigkeit von etwa 8000 U/min etwa 2 Minuten mit 330 Gewichtsteilen einer Ölphase der folgenden Zusammensetzung zusammengerührt:

| | |
|---|---|
| Ölsäure | 50 (Gew.-Teile) |
| Mikrofeines Titandioxid nach Beispiel 1 der EP-B 446 290 (beschichtet mit Lecithin "P Centrolex") | 10 |
| Oleth-5 | 120 |
| Methylglucosediolat (in Gew.-%) | 150 |

Es wurde eine feinteilige Emulsion erhalten, die beim Anrühren mit wäßriger 6%-iger H₂O₂-Lösung eine absolut homogene Mischung ergab.

## Patentansprüche

1. Haarfärbemittel auf Basis einer wäßrigen Emulsion, enthaltend mindestens ein Oxidationsfarbstoff-Vorprodukt, **dadurch gekennzeichnet, daß** die Emulsion ein Gemisch aus
a) 2,5 bis 10 Gew.-% mindestens einer C₁₀-C₁₈-Fettsäure;
b) 5 bis 25 Gew.-% mindestens eines C₁₀-C₂₂-Fettalkoholethoxylats; und
c) 5 bis 30 Gew.-% mindestens eines flüssigen Zuckerfettsäureesters,
jeweils berechnet auf die Gesamtzusammensetzung des Mittels, enthält.

2. Haarfärbemittel nach Anspruch 1, **dadurch gekennzeichnet, daß** es 3 bis 8 Gew.-%, berechnet auf die Gesamtzusammensetzung, mindestens einer C₁₂-C₁₈-Fettsäure enthält.

3. Haarfärbemittel nach Anspruch 1 oder 2, **dadurch gekennzeichnet, daß** es als Fettsäure Ölsäure enthält.

4. Haarfärbemittel nach einem oder mehreren der Ansprüche 1 bis 3, **dadurch gekennzeichnet, daß** es die Bestandteile a) und c) in einem Gewichtsverhältnis von 1 zu 5 bis 1 zu 1 enthält.

5. Haarfärbemittel nach einem oder mehreren der Ansprüche 1 bis 4, **dadurch gekennzeichnet, daß** es den Bestandteil b) in einer Menge von 10 bis 20 Gew.-%, berechnet auf die Gesamtzusammensetzung enthält.

6. Haarfärbemittel nach einem oder mehreren der Ansprüche 1 bis 5, **dadurch gekennzeichnet, daß** es den Bestandteil c) in einer Menge von 10 bis 25 Gew.-%, berechnet auf die Gesamtzusammensetzung enthält.

7. Haarfärbemittel nach einem oder mehreren der Ansprüche 1 bis 6, **dadurch gekennzeichnet, daß** es als Zuckerfettsäureester ein Glucosediolat enthält.

8. Haarfärbemittel nach einem oder mehreren der Ansprüche 1 bis 7, **dadurch gekennzeichnet, daß** es zusätzlich 0,005 bis 1 Gew.-%, berechnet auf die Gesamtzusammensetzung mindestens einer wasserunlöslichen Substanz, ausgewählt aus Titandioxid, Zinkoxid, Talkum, Aluminiumoxid, Wismutoxidchlorid, Calciumcarbonat Magnesiumcarbonat, Calciumsulfat, Calciumphosphaten. Bariumsulfat, Aluminiumsilikat und/oder Alkalialuminiumsilikat enthält.

9. Haarfärbemittel nach Anspruch 8, **dadurch gekennzeichnet, daß** es 0,01 bis 0,5 Gew.-%, berechnet auf die Gesamtzusammensetzung, mindestens einer wasserunlöslichen Substanz d) enthält.

10. Haarfärbemittel nach einem oder mehreren der Ansprüche 8 bis 9, **dadurch gekennzeichnet, daß** es als Bestandteil d) Titandioxid und/oder Zinkoxid enthält.

11. Haarfärbemittel nach Anspruch 10, **dadurch gekennzeichnet, daß** es mit einem Organopolysiloxan oder einem Phospholipid beschichtetes Titandioxid und/oder Zinkoxid enthält.

12. Haarfärbemittel nach einem oder mehreren der Ansprüche 8 bis 11, **dadurch gekennzeichnet, daß** der Bestandteil d) eine mittlere Teilchengröße von weniger als 100 µm aufweist.

13. Haarfärbemittel nach Anspruch 12, **dadurch gekennzeichnet, daß** der Bestandteil d) eine mittlere Teilchengröße von weniger als 1 µm aufweist.

14. Haarfärbemittel nach Anspruch 13, **dadurch gekennzeichnet, daß** der Bestandteil d) eine mittlere Teilchengröße von etwa 30 bis 300 nm aufweist.

15. Verfahren zur Herstellung eines Haarfärbemittels in Form einer stabilen wäßrigen Emulsion, **dadurch gekennzeichnet, daß** etwa 20 bis 40 Gewichtsteile einer Ölphase, enthaltend mindestens eine C₁₂-C₁₈-Fettsäure, mindestens ein C₁₂-C₁₈-Fettalkoholethoxylat und mindestens einen flüssigen Zuckerfettsäureester, mit etwa 80 bis 60 Gewichtsteilen einer wäßrigen Phase, enthaltend mindestens ein Oxidationsfarbstoff-Vorprodukt und mindestens ein wasserlösliches Tensid, bei etwa 15 bis 35 °C vermischt werden.

## Claims

1. Hair dyeing composition on the basis of an aqueous emulsion, comprising at least one oxidation dyestuff precursor, wherein the emulsion contains a mixture of
a) 2.5% to 10% by weight of at least one C₁₀-C₁₈-fatty acid;
b) 5 % to 25% by weight of at least one C₁₀-C₂₂-fatty alcohol ethoxylate; and
c) 5% to 30% by weight of at least one liquid sugar fatty acid ester, each calculated to the total composition.

2. Hair dyeing composition according to claim 1, comprising 3% to 8% by weight, calculated to the total composition, of at least one C₁₂-C₁₈-fatty acid.

3. Hair dyeing composition according to claim 1 or 2, comprising oleic acid as fatty acid.

4. Hair dyeing composition according to one or more of claims 1 to 3, comprising the components a) and c) in a weight proportion from 1 : 5 to 1 : 1.

5. Hair dyeing composition according to one or more of claims 1 to 4, comprising the component b) in an amount from 10% to 20% by weight, calculated to the total composition.

6. Hair dyeing composition according to one or more of claims 1 to 5, comprising the component c) in an amount from 10% to 25% by weight, calculated to the total composition.

7. Hair dyeing composition according to one or more of claims 1 to 6, comprising a glucose diolate as sugar fatty acid ester.

8. Hair dyeing composition according to one or more of claims 1 to 7, comprising additionally 0.005 % to 1 % by weight, of at least one water-insoluble substance selected from titanium dioxide, zinc oxide, talcum, aluminium oxide, bismuth oxide chloride, calcium carbonate, magnesium carbonate, calcium sulfate, calcium phosphates, barium sulfate, aluminium silicate and/or alkali aluminium silicate, each calculated to the total composition.

9. Hair dyeing composition according to claim 8, comprising 0.01 % to 0.5 % by weight, calculated to the total composition, of at least one water-insoluble substance d).

10. Hair dyeing composition according to one or more of claims 8 to 9, comprising titanium dioxide and/or zinc oxide as component d).

11. Hair dyeing composition according to claim 10, comprising titanium oxide and/or zinc oxide coated with a phospholipid.

12. Hair dyeing composition according to one or more of claims 8 to 11, wherein the component d) has an average particle size of less than 100 µm.

13. Hair dyeing composition according to claim 12, wherein the component d) has an average particle size of less than 1 µm.

14. Hair dyeing composition according to claim 13, wherein the component d) has an average particle size of about 30 to 300 nm.

15. Process for the preparation of a stable, aqueous hair dyeing emulsion, wherein about 20 to 40 parts by weight of an oily phase, comprising at least one C₁₂-C₁₈-fatty acid, at least one C₁₂-C₁₈-fatty alcohol ethoxylate, and at least one liquid sugar fatty acid ester, are mixed at about 15° to 35° C with about 80 to 60 parts by weight of an aqueous phase, comprising at least one oxidation dyestuff precursor and at least one water-soluble surfactant.

## Revendications

1. Agent de coloration des cheveux à base d'une émulsion aqueuse, qui contient au moins un précurseur de colorant d'oxydation, **caractérisé en ce que** l'émulsion est constituée d'un mélange de :
a) 2,5 à 10 % en poids d'un acide gras en C₁₀ à C₁₈,
b) 5 à 25 % en poids d'un éthoxylate d'alcool gras en C₁₀ à C₂₂ et
c) de 5 à 30% en poids d'au moins un ester d'acide gras de sucre liquide,
tous ces pourcentages étant calculés par rapport à la composition totale de l'agent.

2. Agent de coloration des cheveux selon la revendication 1, **caractérisé en ce que**, par rapport à la composition totale, il contient de 3 à 8 % en poids d'au moins un acide gras en C₁₂ à C₁₈.

3. Agent de coloration des cheveux selon la revendication 1 ou 2, **caractérisé en ce qu'**il contient de l'acide oléique comme acide gras.

4. Agent de coloration des cheveux selon l'une ou plusieurs des revendications 1 à 3, **caractérisé en ce qu'**il contient les composants a) et c) dans une proportion pondérale comprise entre 1:5 et 1:1.

5. Agent de coloration des cheveux selon l'une ou plusieurs des revendications 1 à 4, **caractérisé en ce qu'**il contient le composant b) en une quantité de 10 à 20 % en poids par rapport à la composition totale.

6. Agent de coloration des cheveux selon l'une ou plusieurs des revendications 1 à 5, **caractérisé en ce qu'**il contient le composant c) en une quantité de 10 à 22 % en poids par rapport à la composition totale.

7. Agent de coloration des cheveux selon l'une ou plusieurs des revendications 1 à 6, **caractérisé en ce qu'**il contient un dioléate de glucose comme ester d'acide gras de sucre.

8. Agent de coloration des cheveux selon l'une ou plusieurs des revendications 1 à 7, **caractérisé en ce qu'**il contient en outre de 0,005 à 1 % en poids, par rapport à la composition totale, d'au moins une substance insoluble dans l'eau, sélectionnée parmi le dioxyde de titane, l'oxyde de zinc, le talc, l'oxyde d'aluminium, l'oxychlorure de bismuth, le carbonate de calcium, le carbonate de magnésium, le sulfate de calcium, les phosphates de calcium, le sulfate de baryum, le silicate d'aluminium et/ou les aluminosilicates d'alcali.

9. Agent de coloration des cheveux selon la revendication 8, **caractérisé en ce qu'**il contient de 0,01 à 0,5 % en poids, par rapport à la composition totale, d'au moins une substance d) insoluble dans l'eau.

10. Agent de coloration des cheveux selon l'une ou plusieurs des revendications 8 à 9, **caractérisé en ce qu'**il contient de l'oxyde de zinc et/ou du dioxyde de titane comme composant d).

11. Agent de coloration des cheveux selon la revendication 10, **caractérisé en ce qu'**il contient de l'oxyde de zinc et/ou du dioxyde de titane revêtu d'un organopolysiloxane ou d'un phospholipide.

12. Agent de coloration des cheveux selon l'une ou plusieurs des revendications 8 à 11, **caractérisé en ce que** le composant d) présente une taille moyenne de particules inférieure à 100 µm.

13. Agent de coloration des cheveux selon la revendication 12, **caractérisé en ce que** le composant d) présente une taille moyenne de particules inférieure à 1 µm.

14. Agent de coloration des cheveux selon la revendication 13, **caractérisé en ce que** le composant d) présente une taille moyenne de particules d'environ 30 à 300 nm.

15. Procédé de préparation d'un agent de coloration des cheveux sous la forme d'une émulsion aqueuse stable, **caractérisé en ce que** l'on mélange, à une température comprise entre 15 et 35°C, de 20 à 40 parties en poids d'une phase huileuse qui contient au moins un acide gras en C₁₂ à C₁₈ et au moins un éthoxylate d'alcool gras en C₁₂ à C₁₈ et au moins un ester d'acide gras de sucre liquide avec environ 80 à 60 parties en poids d'une phase aqueuse qui contient au moins un précurseur de colorant d'oxydation et au moins un agent tensio-actif soluble dans l'eau.
